# FASCICULE DE BREVET EUROPEEN

(11) **EP 2 819 679 B1**
(45) Date de publication et mention de la délivrance du brevet: **16.05.2018**
(21) Numéro de dépôt: 13712305.5
(22) Date de dépôt: 28.02.2013
(51) Int. Cl.: A61K 31/64, A61K 9/10, A61K 47/38, A61K 47/36, A61P 3/10, A61P 27/02

(54) **FORMULATIONS LIQUIDES DE SULFAMIDES HYPOGLYCÉMIANTS**
FLÜSSIGE FORMULIERUNGEN ENTHALTEND HYPOGLYZEMISCHE SULFAMIDE
LIQUID FORMULATIONS COMPRISING HYPOGLYCEMIC SULFAMIDES

(30) Priorité: 28.02.2012 FR 1251795
(43) Date de publication de la demande: 07.01.2015
(73) Titulaire: Ammtek, 75003 Paris (FR)
(72) Inventeur: BERDUGO POLAK, Marianne, 75019 Paris (FR)
(74) Mandataire: Pierru, Bénédicte
(86) Numéro de dépôt international: PCT/FR2013/050421
(87) Numéro de publication internationale: WO 2013/128131

(56) Documents cités:
- WO-A1-2008/015226
- WO-A1-2011/036202
- WO-A2-02/11716
- US-A1- 2005 019 412

## Description

La présente invention relève du domaine des formulations pharmaceutiques, et plus particulièrement de celui des formulations adaptées à un usage pédiatrique, gériatrique ou vétérinaire.

### Arrière-plan technologique de l'invention

Le glibenclamide ou 5-chloro-N-[2-[4-(cyclohexylcarbamoylsulfamoyl)phényl]éthyl]-2-méthoxybenzamide (nomenclature UIPAC) est un inhibiteur des canaux potassiques ATP-dépendants (K_{ATP}) présents à la surface des cellules pancréatiques. Tout comme le glucose, il agit en stimulant la sécrétion d'insuline par les cellules β du pancréas. Ce composé, également connu sous le nom de glyburide (USAN), appartient à la famille des sulfonylurées et est couramment utilisé dans le traitement du diabète de type 2.

Comme tous les sulfamides hypoglycémiants, le glibenclamide est susceptible de provoquer des hypoglycémies pouvant entraîner des convulsions, un coma ou même la mort. Un dosage précis de ce composé en relation avec le poids du patient et/ou l'équilibre glycémique est donc indispensable pour prévenir de tels effets secondaires.

A l'heure actuelle, le glibenclamide n'est disponible que sous la forme de comprimés. Cette forme galénique est totalement inadaptée à l'administration par voie orale à de jeunes enfants ou à des nourrissons, ou encore à des personnes âgées pour lesquelles la prise de comprimés peut être difficile. Pour ces patients, un ou plusieurs comprimés de glibenclamide peuvent être réduits en poudre et la dose nécessaire de poudre est mise en solution dans un liquide, par exemple dans de l'eau. Cependant, ces pratiques conduisent à des variations conséquentes de la quantité de substance active administrée et peuvent également induire des vitesses variables de libération du principe actif en fonction du degré de broyage du comprimé. Ces variations sont essentiellement dues aux incertitudes de la mise en suspension et de la taille particulaire du broyat ainsi qu'à la très faible solubilité du glibenclamide (solubilité dans l'eau à 27°C : 4 mg/L) qui accentue le risque de décantation.

Or, les nourrissons, en particulier les nourrissons prématurés, les jeunes enfants et les personnes âgées sont les populations les plus exposées aux accidents hypoglycémiques. Il est donc capital que la dose administrée à ces patients soit la plus exacte possible.

De plus, les sulfamides hypoglycémiants, notamment le glibenclamide, se sont récemment révélés particulièrement utiles dans le traitement de pathologies oculaires (WO 2011/036202 et WO 2008/015226). Or, il n'existe à ce jour aucune formulation de ces composés adaptée pour une administration par voie ophtalmique, en particulier par application topique.

Par conséquent, il existe un réel besoin pour une formulation liquide de sulfamide hypoglycémiant, et plus particulièrement de glibenclamide, qui soit adaptée à l'administration par voie ophtalmique ou à l'administration par voie orale à des patients pour lesquelles l'utilisation de comprimés n'est pas indiquée, ladite formulation présentant, de préférence, des propriétés organoleptiques acceptables.

### Résumé de l'invention

Les inventeurs ont mis au point une formulation pharmaceutique de sulfamide hypoglycémiant dont les propriétés sont compatibles avec l'administration par voie orale, en particulier à des nourrissons, notamment des nourrissons prématurés, de jeunes enfants ou des personnes pour lesquelles la prise de comprimés peut être difficile, ou avec l'administration par voie ophtalmique.

Ainsi, la présente invention concerne une formulation pharmaceutique liquide comprenant des particules micronisées d'un sulfamide hypoglycémiant, de préférence 90% des particules ayant une taille inférieure à 30 µm, au moins un agent épaississant et un système tampon permettant de maintenir le pH de ladite formulation entre 4 et 8.

La formulation peut comprendre au moins un agent épaississant sélectionné dans le groupe constitué des polysaccharides, des dérivés de cellulose et des carbomères gélifiants, et combinaisons de ceux-ci, de préférence dans le groupe constitué des polysaccharides et des dérivés de cellulose.

La formulation comprend au moins un agent épaississant qui est un dérivé de cellulose, de préférence sélectionné dans le groupe constitué d'un hydroxyalkylcellulose, la méthylcellulose, l'éthylcellulose, l'éthylméthylcellulose, la carboxyméthylcellulose, la carboxyméthylcellulose sodique, et une combinaison de ceux-ci. En particulier, l'hydroxyalkylcellulose peut être sélectionné dans le groupe constitué de l'hydroxyéthylcellulose l'hydroxyméthylcellulose, l'hydroxypropylcellulose et l'hydroxypropylméthylcellulose, et une combinaison de ceux-ci. De manière tout particulièrement préférée, la formulation comprend de l'hydroxyéthylcellulose en tant qu'agent épaississant.

La formulation comprend au moins un agent épaississant qui est un polysaccharide, de préférence sélectionné dans le groupe constitué de la gomme xanthane, la gomme adragante, un carraghénane, un galactomannane, la gomme gellane, la gomme arabique, la gomme karaya, une pectine, de l'amidon et ses dérivés obtenus par estérification ou éthérification, et le tamarin, et une combinaison de ceux-ci. De manière tout particulièrement préférée, la formulation comprend de la gomme xanthane en tant qu'agent épaississant.

La formulation peut comprendre au moins un agent épaississant qui est un carbomère gélifiant, de préférence sélectionné dans le groupe constitué du Carbopol® 934P, du Carbopol® 71G, du Carbopol® 971P et du Carbopol® 974P, et une combinaison de ceux-ci.

De préférence, la formulation comprend de l'hydroxyéthylcellulose et de la gomme xanthane en tant qu'agents épaississants.

La formulation selon l'invention peut comprendre au moins un agent conservateur, de préférence sélectionné dans le groupe constitué de l'acide benzoïque et ses sels sodiques ou potassique, des parabènes, de l'acide sorbique et ses sels sodiques ou potassiques, des ammoniums quaternaires, des dérivés mercuriels, et une combinaison de ceux-ci. De manière tout particulièrement préférée, l'agent conservateur est du benzoate de sodium.

La formulation selon l'invention comprend un système tampon maintenant la formulation à un pH compris entre 4 et 6, composé de citrate de sodium et d'acide lactique.

Le sulfamide hypoglycémiant est le glibenclamide.

La formulation peut comprendre entre 0,01 et 100 mg/mL de sulfamide hypoglycémiant, de préférence entre 0,1 et 10 mg/mL.

Selon un mode préféré, au moins 90% des particules micronisées ont une taille inférieure à 30 µm, de préférence inférieure à 10 µm.

La formulation selon l'invention peut comprendre un mélange d'agents épaississants constitué d'environ 50% d'hydroxyéthylcellulose et d'environ 50% de gomme xanthane.

De préférence, la formulation présente une viscosité comprise entre 350 et 450 mPa.s.

La formulation peut présenter une osmolalité inférieure à 400 mOsm/L, de préférence inférieure à 250 mOsm/L.

Selon un mode préféré, la formulation comprend entre 0,1 et 10 mg/mL de sulfamide hypoglycémiant, entre 8 et 15 mg/mL d'agent(s) épaississant(s), et entre 0,5 et 10 mg/mL d'agent(s) conservateur(s).

En particulier, la formulation peut comprendre entre 0,1 et 10 mg/mL de glibenclamide, environ 5 mg/mL d'hydroxyéthylcellulose, environ 5 mg/mL de gomme xanthane, environ 5 mg/mL de benzoate de sodium, et un système tampon composé de citrate de sodium et d'acide lactique maintenant un pH à environ 4,8.

Elle peut être conditionnée dans un récipient comprenant, ou étant associé à, un système de dispensation volumétrique. Elle peut également être conditionnée dans un récipient unidose.

La formulation peut être destinée à une administration par voie orale, rectale, vaginale ou ophtalmique, en particulier par application topique dans l'oeil. De préférence, la formulation est destinée à une administration par voie orale ou par voie ophtalmique. De manière tout particulièrement préférée, la formulation est destinée à une administration par voie orale.

La formulation selon l'invention peut être utilisée dans le traitement de pathologies chez l'humain ou l'animal. En particulier, la formulation peut être utilisée chez des nourrissons, notamment des nourrissons prématurés, des jeunes enfants, des personnes, en particulier des personnes âgées, pour lesquelles la prise de comprimés est difficile, par exemple des personnes souffrant de troubles de la déglutition.

La pathologie à traiter peut être sélectionnée dans le groupe constitué du diabète de type 2, d'un diabète sucré monogénique tel que le diabète néonatal, un diabète de l'adolescent relevant des mêmes mécanismes moléculaires que ceux du diabète néonatal, un diabète mitochondrial associé à une surdité (MIDD, Maternally Inherited Diabetes and Deafhess) ou un diabète de type MODY (Maturity Onset Diabetes of the Young), l'hyperglycémie néonatale transitoire, un trouble neuropsychologique, musculaire ou neurologique et des pathologies associées à l'ischémie rétinienne et/ou à une excitotoxicité rétinienne. De préférence, la pathologie à traiter est un diabète de type 2, l'hyperglycémie néonatale transitoire ou un diabète néonatal. De manière tout particulièrement préférée, la pathologie à traiter est un diabète de type 2 ou un diabète néonatal.

### Brève description des dessins

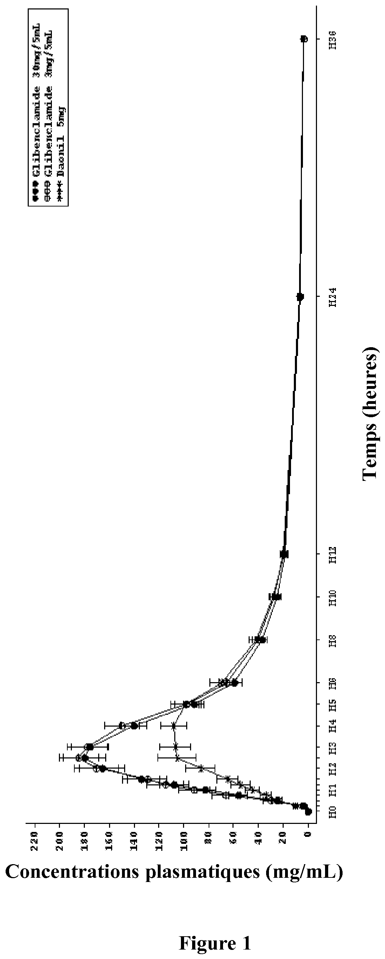

La Figure 1 représente les profils des concentrations moyennes plasmatiques obtenus avec deux formulations de glibenclamide selon l'invention (à 0,6 mg/mL et 6 mg/mL) et un comprimé de Daonil® écrasé dans de l'eau.

### Description détaillée de l'invention

Les inventeurs ont mis au point une formulation liquide de sulfamide hypoglycémiant adaptée à une administration par voie orale ou par voie ophtalmique. Les inventeurs ont notamment développé une formulation qui convient particulièrement à une administration orale dans un usage pédiatrique. Les formulations pharmaceutiques adaptées aux besoins pédiatriques répondent à des critères spécifiques qui ne sont pas nécessairement pris en compte pour les formulations destinées aux adultes. Ainsi, les formulations pédiatriques sont, de préférence, des formulations liquides, avec un goût acceptable, peu ou pas d'odeur, et permettant l'administration de doses précises et variables selon l'âge, le poids et les besoins des jeunes patients. La mise au point d'une formulation liquide pour une administration orale des sulfamides hypoglycémiants est d'autant plus complexe qu'ils sont le plus souvent utilisés dans le traitement de troubles de la glycémie, et que leur formulation ne comprend donc, de préférence, aucune édulcoration à base de sucre ou d'agent édulcorant.

Ainsi, selon un premier aspect, la présente invention concerne une formulation pharmaceutique liquide comprenant des particules micronisées d'un sulfamide, en particulier un sulfamide hypoglycémiant, 90%, ou au moins 90%, des particules ayant une taille inférieure à 30 µm, au moins un agent épaississant, et un système tampon.

Les sulfamides sont des dérivés substitués du sulfamide. Ces composés peuvent avoir notamment une activité antimicrobienne, diurétique ou hypoglycémiante. Les sulfamides hypoglycémiants sont une classe de composés comprenant un groupe sulfonyle lié à un groupe urée. Ils agissent principalement en stimulant la sécrétion d'insuline en se liant à la sous-unité SUR des canaux Kir6.2/SUR1. Ces canaux sont également présents dans le cerveau (Liss et al., 2001) où ils contribuent à la protection du système nerveux central (SNC) contre l'ischémie. Les inhibiteurs de ces canaux présentent également un effet neuroprotecteur rétinien et peuvent donc être utilisés dans le traitement de pathologies rétiniennes, notamment des pathologies associées à l'ischémie ou à des phénomènes d'excitotoxicité rétinienne (WO 2011/036202).

Selon la présente invention, le sulfamide hypoglycémiant est le glibenclamide. Selon un mode particulier de réalisation, le sulfamide hypoglycémiant présente une solubilité dans l'eau à 27°C inférieure à 100 mg/L, de préférence inférieure à 50 mg/L et de manière tout particulièrement préférée inférieure à 25 mg/L. En particulier, le sulfamide hypoglycémiant est le glibenclamide (solubilité dans l'eau à 27°C: 16 mg/L). La formulation selon l'invention est une suspension comprenant des particules micronisées d'un sulfamide hypoglycémiant. Afin d'obtenir une pharmacocinétique satisfaisante, 90 % des particules ont une taille inférieure à 30 µm. Selon un mode particulier de réalisation, 75 % des particules ont une taille inférieure à 10 µm. Selon un mode préféré de 30 réalisation, 90 % des particules ont une taille inférieure à 10 µm.

Les techniques pour obtenir des particules micronisées sont bien connues de l'homme du métier (voir par exemple le brevet EP 0362704). La micronisation peut notamment être obtenue en provoquant des collisions à grande vitesse entre les particules qui induisent leur éclatement en des particules plus petites. Les techniques pour mesurer la taille, ou diamètre, des particules micronisées sont également bien connues de l'homme du métier.

Selon un mode de réalisation particulier, au moins 90 % des particules micronisées ont une taille inférieure à 30 µm, de préférence au moins 95% ou 99%. De manière tout particulièrement préférée, 100 % des particules micronisées ont une taille inférieure à 30 µm.

Selon un autre mode particulier de réalisation, au moins 75 %, de préférence au moins 90, 95 ou 99 %, des particules ont une taille inférieure à 10 µm. De manière tout particulièrement préférée, 100 % des particules micronisées ont une taille inférieure à 10 µm.

Selon un mode de réalisation préférée, au moins 90 %, de préférence au moins 95 ou 99%, des particules micronisées ont une taille inférieure à 30 µm et au moins 75 %, de préférence au moins 90, 95 ou 99%, des particules micronisées ont une taille inférieure à 10 µm.

De préférence, au moins 50% des particules ont une taille inférieure à 5 µm. De manière tout particulièrement préférée, 25%, ou au moins 25%, des particules ont une taille inférieure à 1 µm.

La concentration de sulfamide hypoglycémiant est aisément ajustable par l'homme du métier selon la nature du composé et la population de patients ciblée.

Selon un mode de réalisation, la formulation comprend entre 0,01 et 100 mg/mL de sulfamide hypoglycémiant, de préférence entre 0,05 et 20 mg/mL, de manière plus particulièrement préférée entre 0,1 et 10 mg/mL. Selon un mode particulier de réalisation, la formulation comprend entre 0,1 et 7 mg/mL de sulfamide hypoglycémiant.

Le ou les agents épaississants de la présente formulation permettent d'obtenir une viscosité adéquate afin de maintenir en suspension les particules micronisées du principe actif et de permettre un ajustement posologique précis par mesure volumétrique, par exemple au moyen d'une pipette doseuse.

Selon un mode de réalisation, la formulation comprend un ou plusieurs agents épaississants sélectionnés parmi les dérivés de cellulose. Les dérivés de cellulose utilisables en tant qu'agents épaississants, comprennent, sans y être limités, la méthylcellulose, l'éthylcellulose, l'éthylméthylcellulose, la carboxyméthylcellulose, la carboxyméthylcellulose sodique et des hydroxyalkylcelluloses tels que l'hydroxyéthylcellulose, l'hydroxyméthylcellulose, l'hydroxypropylcellulose et l'hydroxypropylméthylcellulose, et une combinaison de ceux-ci. De manière préférée, la formulation comprend de l'hydroxyéthylcellulose en tant qu'agent épaississant.

La formulation comprend un ou plusieurs agents épaississants sélectionnés parmi les polysaccharides. Les polysaccharides utilisables en tant qu'agent épaississant comprennent, sans y être limités, la gomme xanthane, la gomme adragante, les carraghénanes tels que les λ-carraghénane, le κ-carraghénane ou le t-carraghénane, les galactomannanes tels que la farine de graines de caroube, la farine de graines de guar ou la farine de graines de tara, la gomme gellane, la gomme arabique, la gomme karaya, les pectines, l'amidon et ses dérivés obtenus par estérification ou éthérification, et le tamarin, et une combinaison de ceux-ci. De manière préférée, la formulation comprend de la gomme xanthane en tant qu'agent épaississant.

La formulation peut également comprendre, en tant qu'agents épaississants, un mélange d'un ou plusieurs dérivés de cellulose et d'un ou plusieurs polysaccharides. De préférence, le mélange comprend environ 50% de dérivé(s) de cellulose et environ 50% de polysaccharide(s).

Dans le présent document, le terme « environ » se réfère à une gamme de valeurs de ± 10% de la valeur spécifiée. A titre d'exemple, « environ 50 » comprend les valeurs de ± 10 % de 50, c'est-à-dire les valeurs de la gamme 45 à 55. De préférence, le terme « environ» se réfère à une gamme de valeurs de ± 5% de la valeur spécifiée.

Selon un mode particulier de réalisation, la formulation comprend de l'hydroxyéthylcellulose et de la gomme xanthane en tant qu'agents épaississants, de préférence, environ 50 % d'hydroxyéthylcellulose et environ 50 % de gomme xanthane. De préférence, la formulation ne comprend pas d'autres agents épaississants.

La concentration d'agent(s) épaississant(s) dans la formulation selon l'invention peut varier selon la nature dudit ou desdits agents et selon le mode d'administration envisagé.

Lorsque la formulation est destinée à être administrée par mesure volumétrique, la concentration est, de préférence, choisie de façon à obtenir une viscosité comprise entre 350 et 450 mPa.s, de manière plus particulièrement préférée d'environ 400 mPa.s.

La formulation selon l'invention peut comprendre entre 1 et 50 mg/mL d'agent(s) épaississant(s), de préférence entre 5 et 20 mg/mL, et de manière particulièrement préférée entre 8 et 15 mg/mL. Selon un mode de réalisation particulier, la formulation comprend environ 5 mg/mL d'hydroxyéthylcellulose et environ 5 mg/mL de gomme xanthane.

Afin de prévenir les risques de contamination microbiologique, la formulation selon l'invention comprend, de préférence, au moins un agent conservateur.

Les agents conservateurs utilisables dans la formulation selon l'invention comprennent, sans y être limités, l'acide benzoïque et ses sels sodiques ou potassique tels que le benzoate de sodium ; les parabènes tels que le méthylparabène, le propylparabène ou le butylparabène ; l'acide sorbique et ses sels sodiques ou potassique tels que le sorbate de potassium ; des ammoniums quaternaires tels que le chlorure de benzalkonium ; des dérivés mercuriels tels que les sels de phénylmercure (acétate, borate ou nitrate) ou le thiomersal ; et une combinaison de ceux-ci.

Pour une formulation destinée à une administration par voie orale, l'agent conservateur est de préférence choisi dans le groupe constitué de l'acide benzoïque et ses sels sodiques ou potassique tels que le benzoate de sodium ; les parabènes tels que le méthylparabène, le propylparabène ou le butylparabène ; l'acide sorbique et ses sels sodiques ou potassique tels que le sorbate de potassium ; et une combinaison de ceux-ci.

De préférence, l'agent conservateur est adapté à une utilisation pédiatrique. En raison de risques potentiels sur la fertilité et de leur goût métallique, les parabènes sont de préférence exclus de la présente formulation lorsque celle-ci est destinée à un usage pédiatrique, ou sont utilisés à de très faibles concentrations. Ainsi, selon un mode de réalisation, la formulation est destinée à une administration par voie orale et comprend, en tant qu'agent conservateur, de l'acide benzoïque ou un de ses sels sodiques ou potassique, de préférence du benzoate de sodium, ou de l'acide sorbique ou un de ses sels sodiques ou potassique, de préférence du sorbate de potassium, ou une combinaison de ceux-ci. Selon un mode de réalisation particulier, la formulation comprend de l'acide benzoïque ou un de ses sels sodiques ou potassique, de préférence du benzoate de sodium, en tant qu'agent conservateur. De préférence, la formulation ne comprend pas d'autre agent conservateur.

Pour une formulation destinée à une administration par voie ophtalmique, l'agent conservateur est de préférence choisi dans le groupe constitué des ammoniums quaternaires tels que le chlorure de benzalkonium ; des dérivés mercuriels tels que les sels de phénylmercure (acétate, borate ou nitrate) ou le thiomersal ; et une combinaison de ceux-ci.

La concentration d'agent conservateur peut être aisément ajustée par l'homme du métier, par exemple sur la base des concentrations réglementaires indiquées dans les pharmacopées. Cette concentration peut notamment varier selon l'étanchéité du récipient utilisé, son mode de fermeture ou encore le mode de conservation (au froid ou à température ambiante). De manière particulière, la formulation peut comprendre entre 0,5 à 10 mg/mL d'agent(s) conservateur(s), de préférence environ 5 mg/mL.

La formulation selon l'invention comprend également un agent tampon permettant de maintenir la suspension à un pH prédéterminé compris entre 4 et 6. Le pH de la formulation selon l'invention peut également être choisi de façon à optimiser les effets des agents conservateurs.

De préférence, le système tampon est choisi de façon à préserver un goût neutre. Selon la présente invention, le système tampon est constitué de citrate de sodium et d'acide lactique. De préférence, la formulation comprend entre 5 et 10 mg/mL de citrate de sodium, et de manière plus particulièrement préférée environ 7,5 mg/mL.

La formulation selon l'invention a, de préférence, une osmolalité inférieure à 400 mOsm/L. Selon un mode de réalisation, la formulation selon l'invention a une osmolalité inférieure à 350 mOsm/L, de préférence inférieure à 300 mOsm/L. Selon un mode particulier de réalisation, la formulation est particulièrement adaptée à l'utilisation chez les prématurés et a une osmolalité inférieure à 250 mOsm/L afin de prévenir l'apparition de troubles digestifs.

Bien que les inventeurs aient observé que la formulation selon l'invention présentait un goût acceptable, il est possible d'ajouter une aromatisation. La formulation selon l'invention peut donc comprendre en outre un arôme tel que, par exemple, un arôme fraise, framboise, banane, citron, ou encore caramel.

La formulation peut également comprendre en outre un colorant, notamment afin d'augmenter son acceptabilité vis-à-vis des enfants. De préférence, le colorant est utilisé pour renforcer la crédibilité de l'arôme (par exemple un colorant rose pour un arôme fraise).

Selon un mode préféré de réalisation, la formulation ne comprend ni arôme, ni colorant.

Le diluant utilisé pour la formulation selon l'invention est de préférence choisi pour avoir un goût neutre. Selon un mode préféré de réalisation, le diluant est de l'eau.

Selon un mode particulier de réalisation, la formulation selon l'invention est une formulation pharmaceutique liquide comprenant des particules micronisées de sulfamide hypoglycémiant, de préférence du glibenclamide, 90%, ou au moins 90%, des particules ayant une taille inférieure à 30 µm, de l'hydroxyéthylcellulose et de la gomme xanthane en tant qu'agents épaississants, du benzoate de sodium en tant qu'agent conservateur, et un système tampon composé de citrate de sodium et d'acide lactique.

Selon un mode préféré de réalisation, la formulation selon l'invention est une formulation pharmaceutique liquide comprenant entre 0,1 et 10 mg/mL, de préférence entre 0,5 et 7 mg/mL, de particules micronisées de sulfamide hypoglycémiant, de préférence de glibenclamide, 90%, ou au moins 90%, des particules ayant une taille inférieure à 30 µm, environ 5 mg/mL d'hydroxyéthylcellulose, environ 5 mg/mL de gomme xanthane, environ 5 mg/mL de benzoate de sodium, et un système tampon composé de citrate de sodium et d'acide lactique maintenant un pH à environ 4,8. De préférence, la formulation comprend environ 7,5 mg/mL de citrate de sodium.

La formulation selon l'invention peut être conditionnée dans un récipient multi-dose ou uni-dose. La formulation selon l'invention est, de préférence, conditionnée dans un récipient comprenant, ou étant associé à, un système de dispensation volumétrique tel qu'une pipette doseuse, une seringue doseuse, un compte goutte ou un système délivrant des gouttes unidoses.

Selon un mode préféré de réalisation, la formulation est destinée à être administrée par voie orale.

Selon un autre mode de réalisation, la formulation est destinée à être administrée par voie ophtalmique, de préférence par application topique dans l'oeil.

Selon l'application envisagée et le patient à traiter, la formulation selon l'invention peut également être administrée par voie rectale ou vaginale.

La présente invention concerne également un procédé de préparation de la formulation pharmaceutique selon l'invention comprenant les étapes consistant à :
- mélanger le système tampon et le diluant, de préférence de l'eau ;
- ajouter les particules micronisées et mélanger jusqu'à obtenir une répartition homogène des particules ;
- ajouter le ou les agents épaississants et agiter la préparation jusqu'à l'obtention d'un gel homogène ;
- laisser reposer la préparation jusqu'à l'obtention de la viscosité souhaitée ;
- ajuster le pH, si nécessaire ; et
- ajuster le volume final en ajoutant du diluant.

Optionnellement, si la formulation comprend un agent conservateur, celui-ci est dissout dans le diluant avant ajout du système tampon.

La présente invention concerne également une formulation selon l'invention pour une utilisation dans le traitement de pathologies chez les humains ou les animaux.

En particulier, la formulation selon l'invention est adaptée à une utilisation dans le traitement de pathologies chez des nourrissons, notamment des nourrissons prématurés, des jeunes enfants et des personnes, en particulier des personnes âgées, pour lesquelles la prise de comprimés peut être difficile. La prise de comprimés peut être rendue difficile notamment par des troubles de la déglutition qui sont fréquents chez les personnes âgées et peuvent conduire à des étouffements (fausses routes). Ainsi, la formulation selon l'invention est particulière adaptée à une utilisation dans le traitement de pathologies chez des nourrissons, notamment des nourrissons prématurés, des jeunes enfants, des personnes souffrant de troubles de la déglutition et des personnes âgées.

De préférence, la formulation selon l'invention est utilisée pour traiter des nourrissons et/ou des jeunes enfants. Selon un mode de réalisation, les jeunes enfants ont moins de 12 ans, de préférence moins de 8 ans. Selon un mode de réalisation, les nourrissons ont moins de 2 ans et peuvent être des enfants nés à terme ou des prématurés. De préférence, les nourrissons ont moins d'un an, et de manière tout particulièrement préférée, moins de 8, 6, 4, 2 ou 1 mois. Selon un mode de réalisation particulier, les nourrissons sont des prématurés nés à moins de 32 semaines de grossesse, de préférence à moins de 28 semaines de grossesse.

La formulation selon l'invention peut également être utilisée dans le traitement de pathologies chez des animaux, de préférence des mammifères. Les animaux peuvent être notamment des animaux domestiques ou d'élevage, en particulier des chiens, des chats, des chevaux, des vaches, des moutons, des chèvres, des lapins ou des rongeurs.

La pathologie traitée peut être sélectionnée dans le groupe constitué du diabète de type 2 ; d'un diabète sucré monogénique tel que le diabète néonatal, un diabète de l'adolescent relevant des mêmes mécanismes moléculaires que ceux du diabète néonatal (Sagen et al., 2004 ; Zung et al., 2004 ; Codner et al., 2005 ; Babenko et al., 2006 ; Pearson et al., 2006), un diabète mitochondrial associé à une surdité (MIDD, Maternally Inherited Diabetes and Deafness) ou un diabète de type MODY (Maturity Onset Diabetes of the Young), ; l'hyperglycémie néonatale transitoire, un trouble neuropsychologique, musculaire ou neurologique tel que l'épilepsie, le retard de développement, la faiblesse musculaire, la dyspraxie, la dyslexie, la dystonie, la dysphasie ou encore des troubles oculaires (Zwaveling-Soonawala et al., 2011 ; Slingerland et al., 2008; WO 2008/015226); des pathologies ophtalmologiques, notamment des pathologies associées à l'ischémie rétinienne et/ou à une excitotoxicité rétinienne, telles que le glaucome, des neuropathies optiques glaucomateuses sans hypertonie, de la dégénérescence maculaire liée à l'âge, des inflammations intraoculaires aigues ou chroniques (les uvéites, uvéo-rétinites, les choroïdites), des neuropathies optiques ischémiques ou toxiques, de l'endophtalmie, des rétinites infectieuses, de la rétinopathie diabétique, de la rétinopathie du prématuré, de la rétinopathie ischémique proliférante, des rétinites pigmentaires, des rétinopathies associées à une hémoglobinopathie, d'une photo-dégénérescence, d'un décollement de la rétine, des pathologies vasculaires rétiniennes et choroïdiennes (sténoses, thromboses et occlusions vasculaires), des hémorragies rétiniennes et/ou choroïdiennes, de la myopie et des dégénérescences rétiniennes héréditaires ou acquises (WO 2011/036202).

Selon un mode de réalisation, la pathologie traitée est sélectionnée dans le groupe constitué un diabète de type 2, un diabète néonatal, un diabète de l'adolescent relevant des mêmes mécanismes moléculaires que ceux du diabète néonatal, un diabète mitochondrial associé à une surdité (MIDD), un diabète de type MODY, l'hyperglycémie néonatale transitoire, et un trouble neuropsychologique, musculaire ou neurologique. Selon un mode de réalisation particulier, la pathologie traitée est sélectionnée dans le groupe constitué un diabète de type 2, un diabète néonatal, un diabète de l'adolescent relevant des mêmes mécanismes moléculaires que ceux du diabète néonatal, l'hyperglycémie néonatale transitoire, et un trouble neuropsychologique, musculaire ou neurologique, De préférence, la pathologie traitée est sélectionnée dans le groupe constitué d'un diabète de type 2, un diabète néonatal et l'hyperglycémie néonatale transitoire. De manière tout particulièrement préférée, la pathologie traitée est sélectionnée dans le groupe constitué d'un diabète de type 2 et un diabète néonatal. Selon ce mode de réalisation, la formulation est, de préférence, administrée par voie orale.

Selon un autre mode de réalisation, la pathologie traitée est une pathologie ophtalmologique, de préférence une pathologie associée à l'ischémie rétinienne et/ou à une excitotoxicité rétinienne. Selon ce mode de réalisation, la formulation est, de préférence, administrée par voie ophtalmique, notamment par application topique, c'est-à-dire à la surface de l'oeil.

La présente invention concerne également la formulation pharmaceutique revendiquée pour son utilisation dans une méthode de traitement d'une pathologie telle que définie ci-dessus, comprenant l'administration d'une dose thérapeutiquement efficace de la formulation selon l'invention à un patient souffrant de ladite pathologie, de préférence le patient étant un nourrisson, un jeune enfant, une personne souffrant de troubles de la déglutition ou une personne âgée.

### Exemples d'aspects de l'invention

1. Formulation pharmaceutique liquide comprenant des particules micronisées de glibenclamide, 90% des particules ayant une taille inférieure à 30 µm, au moins un agent épaississant qui est un dérivé de cellulose, au moins un agent épaississant qui est un polysaccharide, et un système tampon maintenant le pH de ladite formulation entre 4 et 6, ledit système tampon étant composé de citrate de sodium a d'acide lactique.
2. Formulation selon l'aspect 1, dans laquelle la formulation comprend au moins un agent épaississant qui est un dérivé de cellulose et au moins un agent épaississant qui est un polysaccharide.
3. Formulation selon l'aspect 1 ou 2, dans laquelle la formulation comprend de l'hydroxyéthylcellulose et de la gomme xanthane en tant qu'agents épaississants.
4. Formulation selon l'un quelconque des aspects précédents, dans laquelle la formulation comprend au moins un agent conservateur, de préférence du benzoate de sodium.
5. Formulation selon l'un quelconque des aspects précédents, dans laquelle la formulation comprend un système tampon maintenant la formulation à un pH compris entre 4,5 et 5,5, de préférence un système tampon composé de citrate de sodium et d'acide lactique.
6. Formulation selon l'un quelconque des aspects précédents, dans laquelle le sulfamide hypoglycémiant est le glibenclamide.
8. Formulation selon l'un quelconque des aspects précédents, dans laquelle 90% des particules micronisées ont une taille inférieure à 10 µm.
9. Formulation selon l'un quelconque des aspects précédents, dans laquelle la formulation présente une viscosité comprise entre 350 et 450 mPa.s.
10. Formulation selon l'un quelconque des aspects précédents, dans laquelle la formulation comprend entre 0,01 et 100 mg/mL de sulfamide hypoglycémiant.
11. Formulation selon l'un quelconque des aspects précédents, dans laquelle la formulation comprend entre 0,1 et 10 mg/mL de glibenclamide, environ 5 mg/mL d'hydroxyéthylcellulose, environ 5 mg/mL de gomme xanthane, environ 5 mg/mL de benzoate de sodium, et un système tampon composé de citrate de sodium et d'acide lactique maintenant un pH à environ 4,8.
12. Formulation selon l'un quelconque des aspects précédents, dans laquelle la formulation est conditionnée dans un récipient comprenant, ou étant associé à, un système de dispensation volumétrique, ou dans un récipient unidose.
13. Formulation selon l'un quelconque des aspects précédents, dans laquelle la formulation est destinée à une administration par voie orale ou par voie ophtalmique.
14. Formulation selon l'un quelconque des aspects précédents pour une utilisation dans le traitement de pathologies chez l'humain, de préférence un nourrisson, un jeune enfant ou une personne pour laquelle la prise de comprimés est difficile, ou chez l'animal.
15. Formulation selon l'aspect 14, dans laquelle la pathologie est sélectionnée dans le groupe constitué du diabète de type 2, du diabète néonatal ou d'un diabète de l'adolescent relevant des mêmes mécanismes moléculaires que ceux du diabète néonatal, un trouble neuropsychologique, musculaire ou neurologique et une pathologie ophtalmologique.

Les exemples ci-dessous sont donnés à titre illustratif et non limitatif.

### Exemples

Les inventeurs ont mis au point une formulation de glibenclamide se présentant sous la forme d'une suspension blanche, inodore et dont le goût est neutre et sans aucune amertume. Cette formulation permet d'envisager son utilisation chez des nourrissons et de jeunes enfants sans avoir à ajouter d'arôme ou de colorant.

### Exemple 1 de formulation selon l'invention :

| Composés | Quantité unitaire pour 5 mL |
|---|---|
| Glibenclamide | 3 mg |
| Hydroxyéthylcellulose (Natrosol 250G Pharm) | 25 mg |
| Gomme xanthane | 25 mg |
| Benzoate de sodium | 25 mg |
| Acide lactique | Qs pH 4,8 |
| Citrate de sodium | 37,5 mg |
| Eau purifiée | Qs 5 mL |

Cette formulation est particulièrement adaptée à une utilisation chez les nourrissons.

### Exemple 2 de formulation selon l'invention :

| Composés | Quantité unitaire pour 5 mL |
|---|---|
| Glibenclamide | 30 mg |
| Hydroxyéthylcellulose (Natrosol 250G Pharm) | 25 mg |
| Gomme xanthane | 25 mg |
| Benzoate de sodium | 25 mg |
| Acide lactique | Qs pH 4,8 |
| Citrate de sodium | 37,5 mg |
| Eau purifiée | Qs 5 mL |

Cette formulation est particulièrement adaptée à une utilisation chez les jeunes enfants.

L'association gomme de xanthane / hydroxyéthylcellulose permet à la fois un bon maintien en suspension du principe actif par la gomme xanthane qui présente un fort pouvoir thixotrope et par l'hydroxyéthylcellulose qui permet de maintenir un niveau de viscosité adapté aux opérations de délivrance par pipette doseuse.

La protection microbiologique de la suspension est réalisée par du benzoate de sodium. Afin de garantir l'efficacité de cet agent de conservation, un système tampon pH a été introduit dans la formulation pour donner à la solution un pH très légèrement acide de 4,8. Le tampon pH est réalisé par un mélange acide lactique / citrate de sodium. Ce couple de tampons a été choisi pour donner un goût le plus neutre possible à la préparation.

S'agissant d'un médicament pédiatrique destiné au traitement des troubles de la glycémie, aucune édulcoration à base de sucre ou d'agent édulcorant n'a été introduite. Malgré cela le produit a un goût neutre sans amertume. Aucune aromatisation n'a été jugée nécessaire pour rendre le produit acceptable par de très jeunes enfants.

Les formulations des exemples 1 et 2 ont été soumises à des tests de stabilité et répondent aux critères de la Pharmacopée Européenne (7ème édition) avec une période de stabilité d'au moins 18 mois.

En particulier, ces formulations se sont révélées conformes aux exigences de la pharmacopée européenne concernant l'uniformité de masse de la dose délivrée de glibenclamide (Ph. Eur. 2.9.27).

### Exemple 3

Trois formulations différentes de glibenclamide ont été administrées par voie orale à 18 volontaires sains adultes après un jeûne de 10 heures durant la nuit. Chaque volontaire a reçu une dose unique de 5 mg de glibenclamide et les concentrations plasmatiques moyennes ont été suivies durant 36 heures. Afin de prévenir tout risque d'hypoglycémie, les volontaires ont reçu une perfusion de 10% glucose 30 minutes avant la prise de glibenclamide et durant 3,5 heures après, à raison de 100mL/h.

La première formulation a été obtenue en écrasant un comprimé de Daonil® comprenant 5 mg de glibenclamide dans de l'eau.

La seconde formulation correspond à la formulation selon l'exemple 1 ci-dessus (3 mg/5mL de glibenclamide, soit 8,33 mL pour 5 mg de glibenclamide).

La troisième formulation correspond à la formulation selon l'exemple 2 ci-dessus (30 mg/5mL de glibenclamide, soit 0,83 mL pour 5 mg de glibenclamide).

Les profils des concentrations moyennes plasmatiques obtenus pour les trois formulations sont présentés dans la figure 1.

Ces résultats montrent tout d'abord que les concentrations plasmatiques maximales obtenues avec les formulations selon l'invention sont presque deux fois supérieures à celle obtenue avec la formulation comprenant le comprimé écrasé.

De plus, les formulations selon l'invention permettent d'obtenir une concentration plasmatique maximale 2,5 heures après l'administration alors que pour la formulation obtenue avec le comprimé écrasé, le pic n'est atteint que 4 heures après l'administration.

Ces résultats démontrent donc que les formulations selon l'invention présentent des propriétés pharmacocinétiques améliorées par comparaison à la formulation obtenue en écrasant un comprimé de Daonil® dans de l'eau.

### Références

Babenko et al. Activating mutations in the ABCC8 gene in neonatal diabetes mellitus. N Engl J Med. 2006 Aug 3;355(5):456-66
Codner, E., et al., High-dose glibenclamide can replace insulin therapy despite transitory diarrhea in early-onset diabetes caused by a novel R201L Kir6.2 mutation. Diabetes Care, 2005. 28(3): p. 758-9.
Liss B, Roeper J. Molecular physiology of neuronal K-ATP channels. Mol. Membr. Biol 2001;18:117-127.
Pearson et al. Switching from insulin to oral sulfonylureas in patients with diabetes due to Kir6.2 mutations.N Engl J Med. 2006 Aug 3;355(5):467-77),
Sagen, J.V., et al., Permanent neonatal diabetes due to mutations in KCNJ11 encoding Kir6.2: patient characteristics and initial response to sulfonylurea therapy. Diabetes, 2004. 53(10): p. 2713-8
Slingerland et al. Sulphonylurea therapy improves cognition in a patient with the V59M KCNJ11 mutation. Diabet Med. 2008 Mar;25(3):277-81
Zung, A., et al., Glibenclamide treatment in permanent neonatal diabetes mellitus due to an activating mutation in Kir6.2. J Clin Endocrinol Metab, 2004. 89(11): p. 5504-7.
Zwaveling-Soonawala et al. Successful transfer to sulfonylurea therapy in an infant with developmental delay, epilepsy and neonataldiabetes (DEND) syndrome and a novel ABCC8 gene mutation. Diabetologia. 2011 Feb;54(2):469-71

## Revendications

1. Formulation pharmaceutique liquide comprenant des particules micronisées de glibenclamide, au moins un agent épaississant qui est un dérivé de cellulose, au moins un agent épaississant qui est un polysaccharide, et un système tampon maintenant le pH de ladite formulation entre 4 et 6, ledit système tampon étant composé de citrate de sodium et d'acide lactique.

2. Formulation selon la revendication 1, **caractérisée en ce que** le dérivé de cellulose est sélectionné dans le groupe constitué d'un hydroxyalkylcellulose, la méthylcellulose, l'éthylcellulose, l'éthylméthylcellulose, la carboxyméthylcellulose, la carboxyméthylcellulose sodique, et une combinaison de ceux-ci.

3. Formulation selon la revendication 1 ou 2, **caractérisée en ce que** le dérivé de cellulose est un hydroxyalkylcellulose sélectionné dans le groupe constitué de l'hydroxyéthylcellulose, l'hydroxyméthylcellulose, l'hydroxypropylcellulose et l'hydroxypropylméthylcellulose, et une combinaison de ceux-ci.

4. Formulation selon l'une quelconque des revendications 1 à 3, **caractérisée en ce que** le polysaccharide est sélectionné dans le groupe constitué de la gomme xanthane, la gomme adragante, un carraghénane, un galactomannane, la gomme gellane, la gomme arabique, la gomme karaya, une pectine, de l'amidon et ses dérivés obtenus par estérification ou éthérification, et le tamarin, et une combinaison de ceux-ci.

5. Formulation selon l'une quelconque des revendications 1 à 4, **caractérisée en ce qu'**elle comprend de l'hydroxyéthylcellulose et de la gomme xanthane en tant qu'agents épaississants.

6. Formulation selon l'une quelconque des revendications précédentes, **caractérisée en ce qu'**elle comprend au moins un agent conservateur.

7. Formulation selon la revendication 6 dans laquelle l'agent conservateur est sélectionné dans le groupe constitué de l'acide benzoïque et ses sels sodiques ou potassiques, des parabènes, de l'acide sorbique et ses sels sodiques ou potassiques, des ammoniums quaternaires, des dérivés mercuriels, et une combinaison de ceux-ci.

8. Formulation selon l'une quelconque des revendications précédentes, **caractérisée en ce qu'**au moins 90% des particules micronisées ont une taille inférieure à 30 µm.

9. Formulation selon la revendication 8, **caractérisée en ce que** 90% des particules micronisées ont une taille inférieure à 10 µm.

10. Formulation selon l'une quelconque des revendications précédentes, **caractérisée en ce qu'**elle comprend entre 0,1 et 10 mg/mL de glibenclamide, entre 8 et 15 mg/mL d'agents épaississants, et entre 0,5 et 10 mg/mL d'agent(s) conservateur(s).

11. Formulation selon l'une quelconque des revendications précédentes, **caractérisée en ce qu'**elle est destinée à une administration par voie orale, ophtalmique, rectale ou vaginale.

12. Formulation selon l'une quelconque des revendications précédentes pour une utilisation dans le traitement de pathologies chez l'humain ou chez l'animal.

13. Formulation selon la revendication 12, pour une utilisation dans le traitement de pathologies chez un humain sélectionné dans le groupe constitué d'un nourrisson, un jeune enfant, une personne souffrant de troubles de la déglutition et une personne âgée.

14. Formulation selon la revendication 13, **caractérisée en ce que** le nourrisson est un nourrisson prématuré.

15. Formulation selon l'une quelconque des revendications 12 à 14, **caractérisée en ce que** la pathologie est sélectionnée dans le groupe constitué du diabète de type 2, un diabète sucré monogénique, l'hyperglycémie néonatale transitoire, un trouble neuropsychologique, musculaire ou neurologique, et une pathologie ophtalmologique.

16. Formulation selon la revendication 15, **caractérisée en ce que** le diabète sucré monogénique est sélectionné dans le groupe constitué du diabète néonatal, un diabète de l'adolescent relevant des mêmes mécanismes moléculaires que ceux du diabète néonatal, un diabète mitochondrial associé à une surdité (MIDD) et un diabète de type MODY.

17. Formulation selon la revendication 15 ou 16, **caractérisée en ce que** la pathologie est sélectionnée dans le groupe constitué d'un diabète de type 2, un diabète néonatal et l'hyperglycémie néonatale transitoire.

## Patentansprüche

1. Flüssige pharmazeutische Formulierung, umfassend mikronisierte Glibenclamid-Partikel, wenigstens ein Verdickungsmittel, das ein Cellulosederivat ist, wenigstens ein Verdickungsmittel, das ein Polysaccharid ist, und ein Puffersystem, das den pH besagter Formulierung zwischen 4 und 6 hält, wobei besagtes Puffersystem aus Natriumcitrat und Milchsäure zusammengesetzt ist.

2. Formulierung gemäß Anspruch 1, **dadurch gekennzeichnet, dass** das Cellulosederivat aus der Gruppe ausgewählt ist, bestehend aus einer Hydroxyalkylcellulose, Methylcellulose, Ethylcellulose, Ethylmethylcellulose, Carboxymethylcellulose, Natriumcarboxymethylcellulose und einer Kombination davon.

3. Formulierung gemäß Anspruch 1 oder 2, **dadurch gekennzeichnet, dass** das Cellulosederivat eine Hydroxyalkylcellulose ist, ausgewählt aus der Gruppe, bestehend aus Hydroxyethylcellulose, Hydroxymethylcellulose, Hydroxypropylcellulose und Hydroxypropylmethylcellulose und einer Kombination davon.

4. Formulierung gemäß einem der Ansprüche 1 bis 3, **dadurch gekennzeichnet, dass** das Polysaccharid aus der Gruppe ausgewählt ist, bestehend aus Xanthangummi, Tragantgummi, einem Carrageen, einem Galactomannan, Gellangummi, Gummi arabicum, Karaya-Gummi, einem Pektin, Stärke und ihren mittels Veresterung oder Veretherung erhaltenen Derivaten, und Tamarinde und einer Kombination davon.

5. Formulierung gemäß einem der Ansprüche 1 bis 4, **dadurch gekennzeichnet, dass** sie Hydroxyethylcellulose und Xanthangummi als Verdickungsmittel umfasst.

6. Formulierung gemäß einem der vorhergehenden Ansprüche, **dadurch gekennzeichnet, dass** sie wenigstens ein Konservierungsmittel umfasst.

7. Formulierung gemäß Anspruch 6, wobei das Konservierungsmittel aus der Gruppe ausgewählt ist, bestehend aus Benzoesäure und ihren Natrium- oder Kaliumsalzen, Parabenen, Sorbinsäure und ihren Natrium- oder Kaliumsalzen, quarternären Ammoniumverbindungen, Quecksilberderivaten und einer Kombination davon.

8. Formulierung gemäß einem der vorhergehenden Ansprüche, **dadurch gekennzeichnet, dass** wenigstens 90% der mikronisierten Partikel eine Größe kleiner als 30 µm besitzen.

9. Formulierung gemäß Anspruch 8, **dadurch gekennzeichnet, dass** 90% der mikronisierten Partikel eine Größe kleiner als 10 µm besitzen.

10. Formulierung gemäß einem der vorhergehenden Ansprüche, **dadurch gekennzeichnet, dass** sie zwischen 0,1 und 10 mg/ml Glibenclamid, zwischen 8 und 15 mg/ml Verdickungsmittel und zwischen 0,5 und 10 mg/ml Konservierungsmittel umfasst.

11. Formulierung gemäß einem der vorhergehenden Ansprüche, **dadurch gekennzeichnet, dass** sie für eine orale, ophthalmische, rektale oder vaginale Verabreichung bestimmt ist.

12. Formulierung gemäß einem der vorhergehenden Ansprüche für eine Verwendung in der Behandlung von Krankheiten bei Mensch und Tier.

13. Formulierung gemäß Anspruch 12 für eine Verwendung in der Behandlung von Krankheiten bei einem Menschen, ausgewählt aus der Gruppe, bestehend aus einem Säugling, einem Kleinkind, einer an Deglutitionsstörungen leidenden Person und einer älteren Person.

14. Formulierung gemäß Anspruch 13, **dadurch gekennzeichnet, dass** der Säugling ein frühgeborener Säugling ist.

15. Formulierung gemäß einem der Ansprüche 12 bis 14, **dadurch gekennzeichnet, dass** die Krankheit aus der Gruppe ausgewählt ist, bestehend aus Diabetes Typ 2, einem monogenen Diabetes mellitus, transitorischer neonataler Hyperglykämie, einer neurologischen, muskulären oder neuropsychologischen Störung und einer ophthalmologischen Krankheit.

16. Formulierung gemäß Anspruch 15, **dadurch gekennzeichnet, dass** der monogene Diabetes mellitus aus der Gruppe ausgewählt ist, bestehend aus neonatalem Diabetes, einem juvenilen Diabetes, bei dem dieselben molekularen Mechanismen wie beim neonatalen Diabetes involviert sind, einem mit einer Taubheit assoziierten mitochondrialen Diabetes (MIDD) und einem Diabetes Typ MODY.

17. Formulierung gemäß Anspruch 15 oder 16, **dadurch gekennzeichnet, dass** die Krankheit aus der Gruppe ausgewählt ist, bestehend aus einem Diabetes Typ 2, einem neonatalen Diabetes und transitorischer neonataler Hyperglykämie.

## Claims

1. A liquid pharmaceutical formulation comprising micronised particles of glibenclamide, at least one thickener which is a cellulose derivative, at least one thickener which is a polysaccharide, and a buffer system maintaining the pH of said formulation between 4 and 6, said buffer system being composed of sodium citrate and lactic acid.

2. The formulation according to claim 1, **characterized in that** the cellulose derivative is selected from the group consisting of a hydroxyalkylcellulose, methylcellulose, ethylcellulose, ethylmethylcellulose, carboxymethylcellulose, sodium carboxymethylcellulose, and a combination thereof.

3. The formulation according to claim 1 or 2, **characterized in that** the cellulose derivative is a hydroxyalkylcellulose selected from the group consisting of hydroxyethylcellulose, hydroxymethylcellulose, hydroxypropylcellulose and hydroxypropylmethylcellulose, and a combination thereof.

4. The formulation according to any one of claims 1 to 3, **characterized in that** the polysaccharide is selected from the group consisting of xanthan gum, tragacanth, a carrageenan, a galactomannan, gellan gum, gum arabic, karaya gum, a pectin, starch and its derivatives obtained by esterification or etherification, and tamarind, and a combination thereof.

5. The formulation according to any one of claims 1 to 4, **characterized in that** it comprises hydroxyethylcellulose and xanthan gum as thickeners.

6. The formulation according to any of the preceding claims, **characterized in that** it comprises at least one preservative.

7. The formulation according to claim 6, **characterized in that** the preservative is selected from the group consisting of benzoic acid and its sodium or potassium salts, parabens, sorbic acid and its sodium or potassium salts, quaternary ammoniums, mercurial derivatives, and a combination thereof.

8. The formulation according to any of the preceding claims, **characterized in that** at least 90% of the micronised particles have a size less than 30 µm.

9. The formulation according to claim 8, **characterized in that** 90% of the micronised particles have a size less than 10 µm.

10. The formulation according to any one of the preceding claims, **characterized in that** it comprises between 0.1 and 10 mg/ml of glibenclamide, between 8 and 15 mg/ml of thickeners, and between 0.5 and 10 mg/ml of preservative(s).

11. The formulation according to any one of the preceding claims, **characterized in that** it is intended for administration by the oral, ophthalmic, rectal or vaginal route.

12. The formulation according to any one of the preceding claims for use in the treatment of diseases in humans or animals.

13. The formulation according to claim 12 for use in the treatment of diseases in a human selected from the group consisting of an infant, a young child, a person suffering from swallowing disorders and an elderly person.

14. The formulation according to claim 13, **characterized in that** the infant is a premature infant.

15. The formulation according to any one of claims 12 to 14, **characterized in that** the disease is selected from the group consisting of type 2 diabetes, monogenic diabetes mellitus, transient neonatal hyperglycemia, a neuropsychological, muscular or neurological disorder, and ophthalmological disease.

16. The formulation according to claim 15, **characterized in that** the monogenic diabetes mellitus is selected from the group consisting of neonatal diabetes, juvenile diabetes involving the same molecular mechanisms as those of neonatal diabetes, mitochondrial diabetes associated with deafness (MIDD) and MODY diabetes.

17. The formulation according to claim 15 or 16, **characterized in that** the disease is selected from the group consisting of type 2 diabetes, neonatal diabetes and transient neonatal hyperglycemia.
